# EUROPÄISCHE PATENTSCHRIFT

(11) **EP 0 732 319 B1**
(45) Veröffentlichungstag und Bekanntmachung des Hinweises auf die Patenterteilung: **14.10.1998**
(21) Anmeldenummer: 96103639.9
(22) Anmeldetag: 08.03.1996
(51) Int. Cl.: C07C 41/42, C07D 223/10, C07D 233/54, C07D 207/26, C07B 63/00

(54) **Verfahren zur Aufarbeitung von in Additionsreaktionen an Acetylen oder Propin anfallenden Reaktionsgemischen**
Process for working-up reaction mixtures formed by addition reactions of acetylene or propyne
Procédé de traitement de mélanges réactionnels obtenus par réactions d'addition d'acétylène ou de propyne

(30) Priorität: 15.03.1995 DE 19509352
(43) Veröffentlichungstag der Anmeldung: 18.09.1996
(73) Patentinhaber: BASF AKTIENGESELLSCHAFT, 67056 Ludwigshafen (DE)
(72) Erfinder: Heider, Marc, Dr., 67433 Neustadt (DE); Karcher, Michael, Dr., 68723 Schwetzingen (DE); Schmidt-Radde, Martin, Dr., 67259 Beindersheim (DE); Dams, Albrecht, Dr., 67157 Wachenheim (DE)

(56) Entgegenhaltungen:
- WO-A-91/05756
- DE-A- 1 812 602
- DE-A- 3 215 093
- US-A- 2 806 847
- RESEARCH DISCLOSURE, Nr. 290, Juni 1988, HAVANT GB, Seite 389 XP002005399 "Distilling organic compounds in the presence of a polyethylene glycol distillation aid"

## Beschreibung

Die vorliegende Erfindung betrifft ein neues Verfahren zur Aufarbeitung von in Additionsreaktionen von OH- oder NH-Gruppen tragenden Verbindungen an Acetylen oder Propin in Gegenwart von Alkalimetallalkoholaten oder -amiden anfallenden Reaktionsgemischen.

Die Addition von OH- oder NH-Gruppen tragenden Verbindungen an Acetylen oder Propin unter Bildung von Vinylethern bzw. N-Vinylverbindungen ist an sich bekannt (z.B. Liebigs Ann. Chem. 601 (1956) 81). Als Katalysatoren dienen dabei Alkalimetallalkoholate bzw. -amide. Die in diesen Reaktionen anfallenden Reaktionsgemische enthalten destillierbare Verbindungen wie die gewünschten Additionsprodukte sowie gegebenenfalls noch vorhandene Ausgangsverbindungen, weiterhin aber nicht destillierbare Verbindungen, zu denen die Katalysatoren sowie polymere Nebenprodukte der Reaktion zählen.

Eine einfache Abtrennung, insbesondere eine für technische Verfahren wünschenswerte kontinuierliche Abtrennung des Verfahrensproduktes ist dann möglich, wenn das Verfahrensprodukt unter den Reaktionsbedingungen gasförmig vorliegt. Durch diese Randbedingung ist eine solche Abtrennung aber auf wenige Verfahrensprodukte beschränkt (Ullmanns Encyklopädie der technischen Chemie, 4. Auflage, Verlag Chemie 1983, Bd. 23, S. 608 ff).

Aus der WO-A 91/05756 ist ein Verfahren zur Addition von propoxyliertem Glycerin an Propin und Acetylen bekannt, bei dem die erhaltenen Trivinylether mit Hilfe eines Dünnschichtverdampfers abdestilliert werden. Die Abtrennung der Trivinylether ist jedoch unbefriedigend.

Bei der diskontinuierlichen Destillation der Reaktionsgemische verbleiben die nicht destillierbaren Anteile im Sumpf der Destillationsblase und müssen nach beendeter Destillation mit geeigneten Lösungsmitteln verdünnt und entsorgt werden (loc. cit.).

Es bestand die Aufgabe, ein Verfahren bereitzustellen, das auf eine Vielzahl von verschiedenen Reaktionsgemischen anwendbar ist, die bei Additionsreaktionen an Acetylen und Propin anfallen. Insbesondere sollte ein Verfahren gefunden werden, das eine kontinuierliche Aufarbeitung solcher Reaktionsgemische erlaubt.

Demgemäß wurde das oben definierte Verfahren gefunden, bei dem die flüchtigen Komponenten in einem Dünnschichtverdampfer aus dem Reaktionsgemisch abdestilliert werden, daß dadurch gekennzeichnet ist, daß man dem Reaktionsgemisch vor der Destillation Polyether der allgemeinen Formel I

R¹― O(̵A―O)̵ₙR² I

in der R¹ und R² für Wasserstoff, einen Alkyl-, Cycloalkyl- oder Arylrest stehen, A für einen geradkettigen oder verzweigten C₂-C₆-Alkylenrest und n für eine ganze Zahl steht, mit einem Molekulargewicht von 200 bis 10.000 g/mol in Mengen von 0,1 bis 20 Gew.-%, bezogen auf das Reaktionsgemisch, zusetzt.

Das erfindungsgemäße Verfahren läßt sich auf eine Vielzahl von Reaktionsgemischen anwenden. Diese Reaktionsgemische fallen bei Additionsreaktionen von OH- oder NH-Gruppen tragenden Verbindungen an Acetylen oder Propin an. Im einzelnen können Alkohole wie Alkanole, bevorzugt C₁-C₂₀-Alkanole, z.B. Methanol, Ethanol, n-Propanol, iso-Propanol, n-Butanol, iso-Butanol, sec.-Butanol, 2-Ethylhexanol, Dodecanol und Octadecanol, nichtaromatische Diole, bevorzugt C₂-C₂₀-Diole wie Ethylenglykol, Propylenglykol, 1,4-Butandiol, 1,6-Hexandiol, 1,4-Cyclohexandimethanol oder Triole wie Trimethylolpropan addiert werden. Als NH-Gruppen tragende Verbindungen kommen in Betracht: C₄-C₁₀-Lactame wie Pyrrolidon, Piperidon und Caprolactam, C₂-C₂₀-Amide wie N-Methylacetamid, Heteroarylverbindungen wie Pyrrol, Imidazol, 2-Methylimidazol, Indol, Carbazol, weiterhin Ethylenharnstoff sowie sekundäre aliphatische Amine.

Bevorzugt sind hiervon Octadecanol, Butandiol, Caprolactam, Carbazol, Pyrrolidon, Imidazol, 2-Methylimidazol und Ethylenharnstoff.

Von den Alkinen ist Acetylen bevorzugt.

Die Addition der genannten OH- oder NH-Gruppen tragenden Verbindungen wird in Gegenwart von Katalysatoren vorgenommen. Hierbei werden bevorzugt die Alkalimetallsalze der zu addierenden OH- oder NH-Gruppen tragenden Verbindungen verwendet. Diese Katalysatoren können vorteilhaft durch Zugabe eines Alkalimetallhydroxids zur Mischung der Ausgangsverbindungen in situ hergestellt werden.

Die Addition wird im allgemeinen bei Temperaturen von 50 bis 250°C und Drücken von 1 bis 25 bar über 1 bis 10 Stunden vorgenommen. Gegebenenfalls wird in einem inerten Lösungsmittel, z.B. einem polaren aprotischen Lösungsmittel wie N-Methylpyrrolidon gearbeitet; bevorzugt ist jedoch eine lösungsmittelfreie Umsetzung. Nach Beendigung der Umsetzung wird das so erhaltene Reaktionsgemisch bevorzugt entgast.

Die Reaktionsgemische enthalten im allgemeinen 20 bis 95 Gew.-%, bevorzugt 85 bis 95 Gew.-% an Verfahrensprodukt, gegebenenfalls Ausgangsverbindungen sowie gegebenenfalls Lösungsmittel, weiterhin im allgemeinen 5 bis 80 Gew.-%, bevorzugt 5 bis 15 Gew.-% Katalysator, Zersetzungsprodukte des Katalysators und polymere Nebenprodukte.

Erfindungsgemäß werden alle flüchtigen Bestandteile nach erfolgtem Zusatz des Polyethers der allgemeinen Formel I in einem Dünnschichtverdampfer aus dem Reaktionsgemisch abdestilliert. Das Reaktionsgemisch wird dazu als dünnen Film auf eine beheizte Fläche gebracht. Solche Dünnschichtverdampfer sind an sich bekannt und im Handel erhältlich. Als vorteilhaft haben sich solche Verdampfer erwiesen, in denen ein großblättriger Rührer das Reaktionsgemisch kontinuierlich auf die beheizte Außenfläche als dünnen Film aufbringt. Die Temperatur des Verdampfers richtet sich nach der Siedetemperatur der höchstsiedenden Komponente im Reaktionsgemisch. Der Druck bei der Destillation kann je nach Verfahrensprodukt 1 mbar bis 2 bar betragen. In der Regel gehen die flüchtigen Komponenten über Kopf. Sie können gegebenenfalls weiter gereinigt werden, beispielsweise destillativ. Die nicht destillierbaren Komponenten können als viskose Masse am Boden des Verdampfers entnommen werden.

Das beschriebene Verfahren ermöglicht die kontinuierliche Aufarbeitung von Reaktionsgemischen, die bei der Addition von OH- oder NH-Gruppen tragenden Verbindungen an Acetylen oder Propin unter Verwendung von Alkalimetallalkoholaten oder -amiden als Katalysator anfallen.

Dem Reaktionsgemischen werden erfindungsgemäß vor der Destillation bevorzugt 1 bis 10 Gew.-% eines Polyethers der Formel I mit einem Molekulargewicht von vorzugsweise 400 bis 2.000 g/mol zugesetzt.

Die Variablen R¹ und R² in der allgemeinen Formel I stehen unabhängig voneinander bevorzugt für Wasserstoff, aber auch Alkylgruppen, bevorzugt C₁-C₆-Alkyl, Cycloalkylgruppen, bevorzugt C₄-C₇-Cycloalkyl, und Arylgruppen, bevorzugt Phenyl kommen in Betracht. Der Rest A steht für einen geradkettigen oder verzweigten Alkylenrest. Die Variable n steht für eine ganze Zahl, die je nach den Resten A so gewählt werden kann, daß die Verbindung der Formel I das oben angegebene Molekulargewicht aufweist.

Die Polyether der Formel I sind polymere Verbindungen, die sich von cyclischen Ethern wie Ethylenoxid, 1,2-Propylenoxid, 1,3-Propylenoxid oder Tetrahydrofuran ableiten. Sie sind in bekannter Weise durch basenkatalysierte Polymerisation in Gegenwart von Wasser oder eines Alkohols erhältlich. Die Polyether I können gleiche oder verschiedene Reste A enthalten, wobei verschiedene Reste A statistisch verteilt oder in Blöcken angeordnet sein können. (s. Ullmanns Encyklopädie der Technischen Chemie, 4. Auflage, Verlag Chemie, Bd. 19, S. 31ff). Die Molgewichtsverteilung der Polyether ist an sich nicht kritisch, jedoch sollten größere Mengen kurzkettiger Verbindungen vermieden werden, da diese mit den flüchtigen Komponenten aus dem Reaktionsgemisch ausgetragen werden können.

Bevorzugt wird Polyethylenglykol als Zusatz zu den Reaktionsgemischen verwendet.

Die Polyether der Formel I sind problemlos mit den Reaktionsgemischen vermischbar. Unter den Destillationsbedingungen werden sie nicht zersetzt.

Die genannten Polyether bewirken eine Erniedrigung der Viskosität der nicht destillierbaren Anteile der Reaktionsgemische. Die nicht destillierbaren Rückstände fließen dadurch leichter aus dem Verdampfer ab und eine Verstopfung durch Ablagerung der Rückstände im Verdampfer wird wirkungsvoll vermieden.

### Beispiele

In einen beheizten Dünnschichtverdampfer mit Rührblättern wurde kontinuierlich ein konstanter Strom verschiedener Reaktionsgemische eingespeist. Der destillierbare Anteil wurde kondensiert (Destillat), der Sumpfablauf wurde am Boden des Verdampfers entnommen und gesammelt (Rückstand).

Der destillierbare Anteil im Rückstand wurde durch Erhitzen des Rückstands auf über 200°C bei ca. 1 mbar bestimmt.

Als Zusatz wurde in den erfindungsgemäßen Beispielen 1, 3, 5, 6, 8 und 9 ein Polyethylenglykol mit einem Molekulargewicht von 600 g/mol verwendet, in Beispiel 10 ein Polypropylenglykol mit einem Molekulargewicht von 600 g/mol und in Beispiel 11 ein Polytetrahydrofuran mit einem Molekulargewicht von 650 g/mol.

Das Reaktionsgemisch wurde jeweils durch basenkatalysierte Umsetzung aus Acetylen und der entsprechenden OH- oder NH-Gruppen enthaltenden Verbindung hergestellt (zur Charakterisierung wird das jeweilige Verfahrensprodukt angegeben).

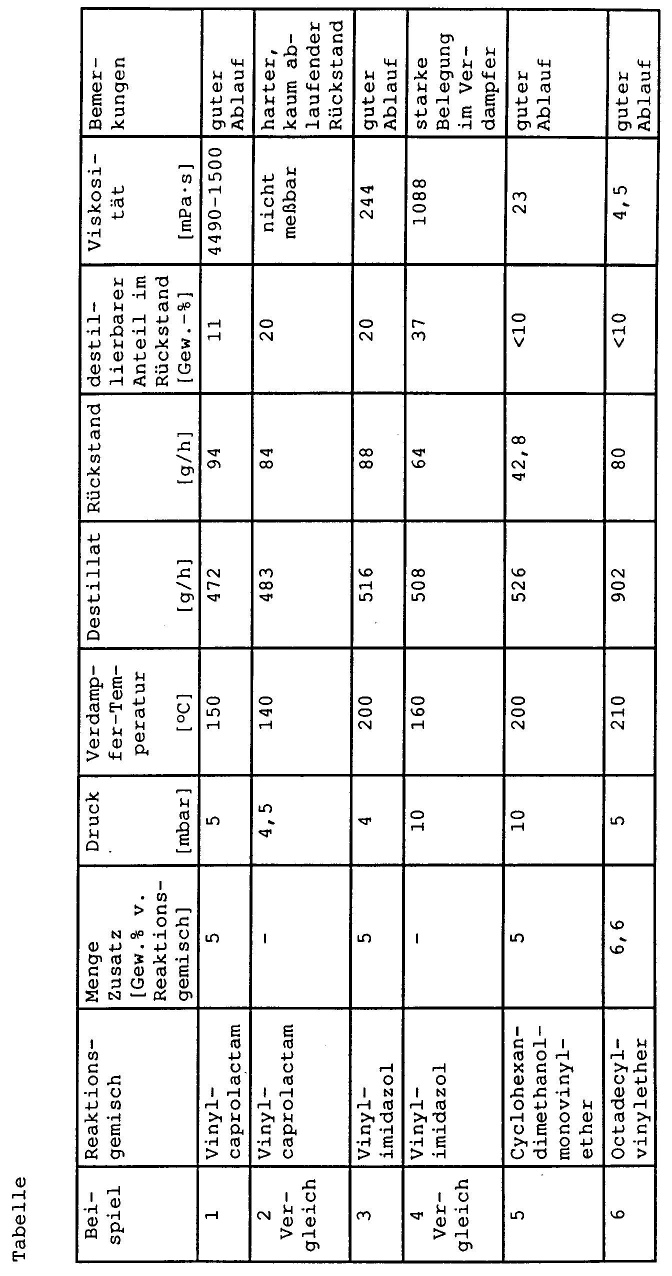

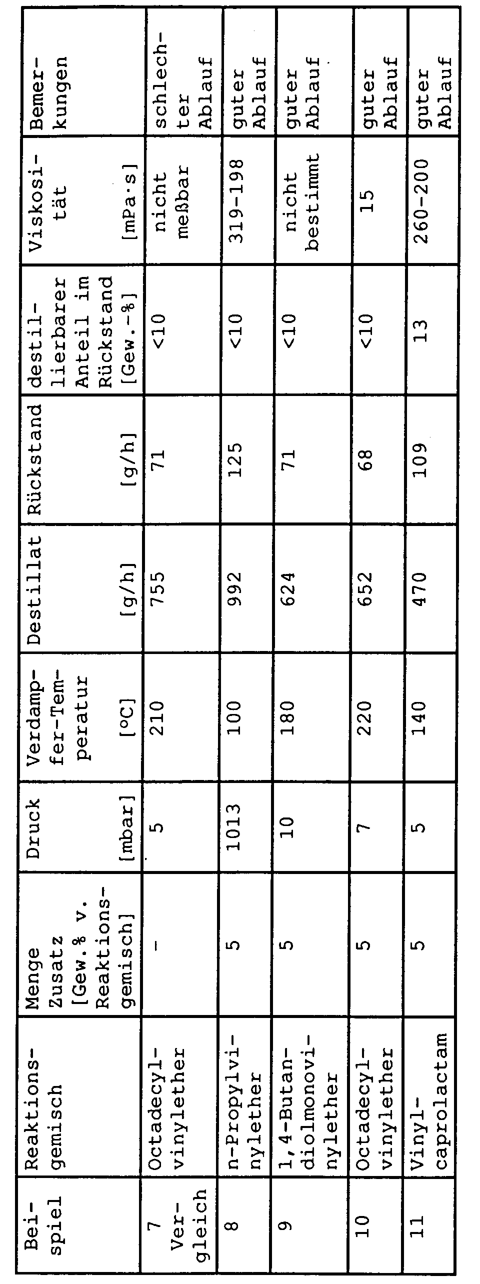

## Patentansprüche

1. Verfahren zur Aufarbeitung von in Additionsreaktionen von OH- oder NH-Gruppen tragenden Verbindungen an Acetylen oder Propin in Gegenwart von Alkalimetallalkoholaten oder -amiden anfallenden Reaktionsgemischen, bei dem die flüchtigen Komponenten in einem Dünnschichtverdampfer aus dem Reaktionsgemisch abdestilliert werden, dadurch gekennzeichnet, daß man dem Reaktionsgemisch vor der Destillation Polyether der allgemeinen Formel I
R¹― O(̵A―O)̵ₙR² I
in der R¹ und R² für Wasserstoff, einen Alkyl-, Cycloalkyl- oder Arylrest stehen, A für einen geradkettigen oder verzweigten C₂-C₆-Alkylenrest und n für eine ganze Zahl steht, mit einem Molekulargewicht von 200 bis 10.000 g/mol in Mengen von 0,1 bis 20 Gew.-%, bezogen auf das Reaktionsgemisch, zusetzt.

2. Verfahren nach Anspruch 1, dadurch gekennzeichnet, daß man ein bei der Addition von Octadecanol, Butandiol, Caprolactam, Carbazol, Pyrrolidon, Imidazol, 2-Methylimidazol oder Ethylenharnstoff an Acetylen anfallendes Reaktionsgemisch aufarbeitet.

3. Verfahren nach Anspruch 1 oder 2, dadurch gekennzeichnet, daß man ein Polyethylenglykol der Formel I mit einem Molgewicht von 400 bis 2.000 g/mol verwendet.

4. Verfahren nach Anspruch 1 bis 3, dadurch gekennzeichnet, daß man das Verfahren kontinuierlich ausübt.

## Claims

1. A process for working up reaction mixtures obtained in addition reactions of OH- or NH-carrying compounds with acetylene or propyne in the presence of alkali metal alcoholates or alkali metal amides, in which the volatile components are distilled off from the reaction mixture in a thin-film evaporator, wherein a polyether of the formula I
R¹― O(̵A―O)̵ₙR² I
where R¹ and R² are each hydrogen, alkyl, cycloalkyl or aryl, A is straight-chain or branched C₂-C₆-alkylene and n is an integer, and having a molecular weight of from 200 to 10,000 g/mol is added to the reaction mixture in an amount of from 0.1 to 20% by weight, based on the reaction mixture, before the distillation.

2. A process as claimed in claim 1, wherein a reaction mixture obtained in the addition reaction of octadecanol, butanediol, caprolactam, carbazole, pyrrolidone, imidazole, 2-methylimidazole or ethyleneurea with acetylene is worked up.

3. A process as claimed in claim 1 or 2, wherein a polyethylene glycol of the formula I having a molecular weight of from 400 to 2,000 g/mol is used.

4. A process as claimed in any of claims 1 to 3, wherein the process is carried out continuously.

## Revendications

1. Procédé de traitement de produits réactionnels obtenus lors de la réaction d'addition de composés portant des groupements OH ou NH sur de l'acétylène ou du propyne en présence d'alcoolates ou d'amides de métaux alcalins, dans lequel les constituants volatils sont éliminés du mélange réactionnel par distillation dans un évaporateur à couche mince, caractérisé en ce que l'on ajoute au mélange réactionnel, avant la distillation, à raison de 0,1-20% en poids, par rapport au mélange réactionnel, un polyéther de formule générale I
R¹― O(̵A―O)̵ₙR² I
dans laquelle R¹ et R² sont mis pour des atomes d'hydrogène, un reste alkyle, cycloalkyle ou aryle, A est mis pour un reste alkylène en C₂-C₆ linéaire ou ramifié et n pour un nombre entier, avec une masse moléculaire de 200-10 000 g/mol.

2. Procédé selon la revendication 1, caractérisé en ce que l'on traite un mélange réactionnel obtenu par addition d'octadécanol, de butanediol, de caprolactame, de carbazole, de pyrrolidone, d'imidazole, de 2-méthylimidazole ou d'éthylène-urée sur de l'acétylène.

3. Procédé selon la revendication 1 ou 2, caractérisé en ce que l'on utilise un polyéthylèneglycol de formule I ayant une masse molaire de 400-2 000 g/mol.

4. Procédé selon l'une quelconque des revendications 1 à 3, caractérisé en ce que l'on met en oeuvre le procédé de façon continue.
